# EUROPEAN PATENT APPLICATION

(11) **EP 4 840 475 A1**
(43) Date of publication of application: **18.11.2026**
(21) Application number: 25175592.2
(22) Date of filing: 12.05.2025
(51) Int. Cl.: A61K 51/04

(54) **METHOD FOR PREPARING RADIONUCLIDE COMPLEX**

(71) Applicant: National Atomic Research Institute, Taoyuan 32546 (TW)
(72) Inventor: LO, Shih-Wei, 32546 Taoyuan City (TW); LEE, Shih-Ying, 32546 Taoyuan City (TW); WANG, Chen-Yu, 32546 Taoyuan City (TW); LU, An-Chi, 32546 Taoyuan City (TW); CHANG, Po-Chih, 32546 Taoyuan City (TW); LIN, Yun-Sheng, 32546 Taoyuan City (TW)
(74) Representative: Lermer, Christoph

(57) **Abstract**

The present disclosure relates to a method for preparing a radionuclide complex, where the radionuclide complex has a structure as represented by formula (1). The preparation method includes the following steps: (a) mixing NARI-PSMA and an ascorbic acid buffer solution to form a mixed solution; (b) adding a radionuclide to the mixed solution to obtain a mixed solution containing the radionuclide; and (c) heating the mixed solution containing the radionuclide at 90°C-130°C for 10-30 minutes to obtain the compound of formula (1).

## Description

### BACKGROUND

### Technical Field

The present invention relates to the field of radiological medicine, and particularly to a method for preparing a complex labeled with a radionuclide.

### Related Art

A variety of methods have been developed for the preparation technologies of radiopharmaceuticals, including general laboratory operation methods, microwave reactor technologies, fully automatic synthesis modules (such as the GRP system, Scintomics, Germany), cold kit technologies, systematic methods and freeze-drying technologies, etc. These methods are generally used to synthesize radiolabeled compounds of target molecules such as DOTATATE, DOTATOC, PSMA-11, PSMA-I&T, PSMA-617 and DOTA-EB-PSMA.

However, the radiolabeling processes of different target molecules have different requirements for reaction conditions (such as temperature, pH value, types and concentrations of reagents), thus putting forward higher requirements of flexibility and precision for the preparation methods. The related art is generally too cumbersome and time-consuming when preparing these radiolabeled complexes. The preparation process may involve multi-step chemical reactions, long waits or separations, and the overall process may take several hours to complete.

The limitations of the above-mentioned technologies are particularly significant in clinical applications. Since radionuclides have the characteristic of a short half-life, the preparation efficiency and operational convenience of the related art can hardly meet clinical needs. In addition, the complicated operation process imposes relatively high requirements on the professional skills of operators and the precision of equipment, increasing costs and operational risks. Therefore, an improved preparation method is urgently needed in the art to improve the preparation efficiency and operational convenience of radionuclide-labeled compounds, so as to overcome the defects in the related art and meet the actual clinical needs.

### SUMMARY

In order to enable readers to understand the basic meaning of the present disclosure, the summary provides a brief description of the present disclosure. The summary is not a complete description of the present disclosure, and it is not intended to define the technical features or the scope of rights of the present invention.

An aspect of the present invention relates to a method for preparing a radionuclide complex, where the radionuclide complex has a structure as represented by formula (1), where X is a radionuclide: 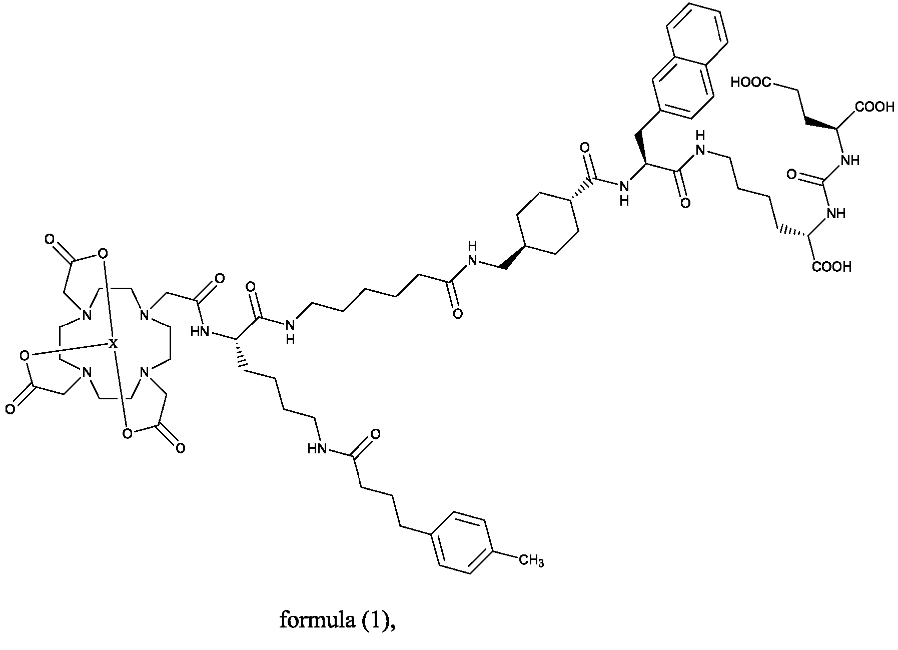 and the method comprises:
(a) mixing NARI-PSMA and an ascorbic acid buffer solution to form a mixed solution, where the NARI-PSMA is as represented by a compound of formula (2): 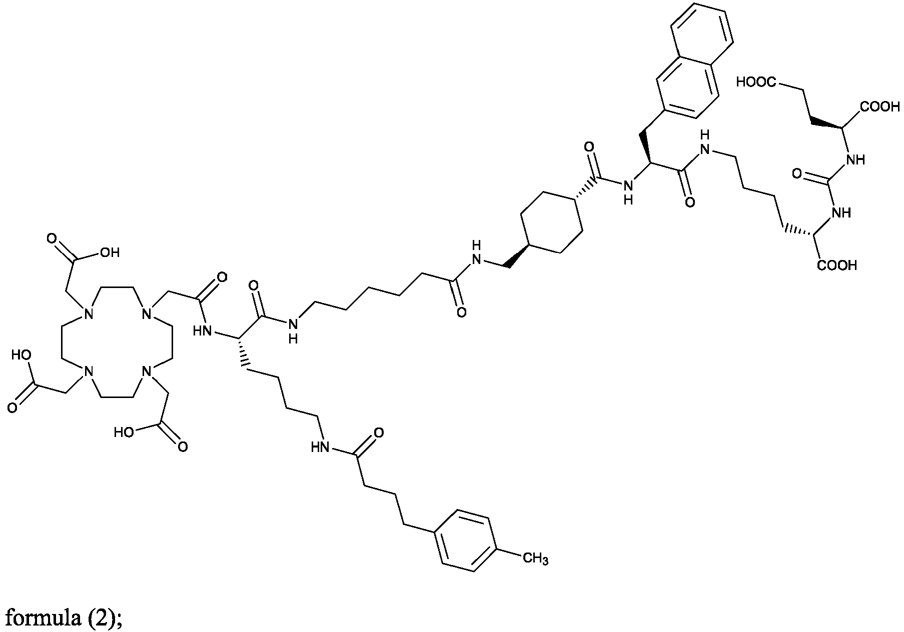
(b) adding a radionuclide to the mixed solution to obtain a mixed solution containing the radionuclide; and
(c) heating the mixed solution containing the radionuclide at 90°C-130°C for at least 10-30 minutes to obtain the compound of formula (1).

In an optional embodiment, a concentration of the NARI-PSMA in the mixed solution is 0.0315-0.049 M.

In an optional embodiment, in the method according to the present invention, the X in the compound of formula (1) is a radionuclide and may be Lu-177, Ac-225, Cu-64 or Cu-67. Furthermore, in a non-limiting embodiment, concentrations of these radionuclides are 100-200 mCi for Lu-177, 0.1-0.4 mCi for Ac-225, 5-20 mCi for Cu-64, and 100-200 mCi for Cu-67.

According to an embodiment of the present invention, the step (c) of the method of the present invention may further comprise filtering the mixed solution using a filter membrane to remove contamination sources and maintain a sterile state. Therefore, a pore diameter of the filter membrane is preferably 0.22 µm or less.

Furthermore, in the step (c), an ion exchange column may also be used to purify the mixed solution.

In addition, according to another embodiment of the present invention, the mixed solution in the step (a) may also be freeze-dried to obtain a lyophilized formulation, and in the step (b), water and the radionuclide are added to the mixed solution to obtain the mixed solution containing the radionuclide.

According to a specific embodiment of the present invention, the ascorbic acid buffer solution has a pH value of 4.0-5.0. In a non-limiting embodiment, a concentration of ascorbic acid in the ascorbic acid buffer solution is 0.02-0.72 M. In another embodiment, the ascorbic acid buffer solution mainly includes ascorbic acid and sodium hydroxide. In an embodiment, a concentration of the ascorbic acid is about 0.28 M, and a concentration of the sodium hydroxide is about 0.20 M. In other embodiments, a concentration of the ascorbic acid is 45-55 mg/mL, and a concentration of the sodium hydroxide is 7.11-8.69 mg/mL.

The steps (a) to (c) disclosed in the method of the present invention may be performed using an auto-synthesizer.

Furthermore, the method of the present invention may also be performed using a general experimental operation mode, and in the step (c), the heating is performed through a sterilization autoclave. In an embodiment, in the step (c), the mixed solution containing the radionuclide is heated to 121°C for at least 130 minutes to obtain the compound of formula (1).

Those having ordinary skill in the art to which the present invention belongs may fully understand the central concept of the present invention, the technical means adopted and various implementation aspects after referring to the detailed description below.

### BRIEF DESCRIPTION OF THE DRAWINGS

In order to make the above and other objectives, features, advantages and examples of the present invention more apparent and understandable, the drawings are described as follows:
FIG. 1 is a flowchart of preparing a radionuclide complex having a structure of formula (1) using an auto-synthesizer according to an embodiment of the present invention;
FIG. 2 is a flowchart of preparing a radionuclide complex having a structure of formula (1) in the form of a lyophilized formulation according to an embodiment of the present invention; and
FIG. 3 is a flowchart of preparing a radionuclide complex having a structure of formula (1) using a sterilization autoclave according to an embodiment of the present invention.

### DETAILED DESCRIPTION

In order to make the description of the present disclosure more detailed and complete, the following presents illustrative textual descriptions with respect to the implementation aspects and specific examples of the present invention. However, the implementation aspects and specific examples of the present invention are not limited thereto.

Unless otherwise stated, the meanings of scientific and technical terms used in this specification are the same as those understood and commonly used by those having ordinary skill in the art. Furthermore, the nouns used in this specification encompass both the singular and plural forms of the nouns, unless otherwise specified.

In this specification, the term "about" generally means that the actual value is within plus or minus 10%, 5%, 1% or 0.5% of a particular value or range. The term "about" herein represents that the actual value falls within the acceptable standard error of the mean value, depending on the consideration of those having ordinary skill in the art to which the present invention belongs. Except for experimental examples, or unless otherwise explicitly stated, it should be understood that the ranges, quantities, values and percentages used herein are all modified by "about". Therefore, unless otherwise stated, the numerical values or parameters disclosed in this specification and the accompanying claims are all approximate numerical values and may vary as required.

The term "individual" refers to animals including humans, i.e. animals to which the radionuclide complex of formula (1) is applicable. Unless specifically indicated, the term "individual" means both males and females.

In order to solve the problems of the related art, the objective of the present invention is to provide a method for preparing a radionuclide complex. The preparation method is fast and convenient and can improve the defects of the related art.

In the preparation method of the present invention, a PSMA-targeting radiopharmaceutical complex is prepared mainly through mixing an ascorbic acid molecule with a PSMA-targeting molecule, and heating a reaction solution containing an ascorbic acid solution, a PSMA-targeting molecule and a radionuclide. The steps may be realized by any of the following ways, including: an automatic synthesis kit, a lyophilized formulation or a sterilization autoclave.

The radionuclide complex of the present invention is a PSMA-targeting molecule having a structure of formula (1):

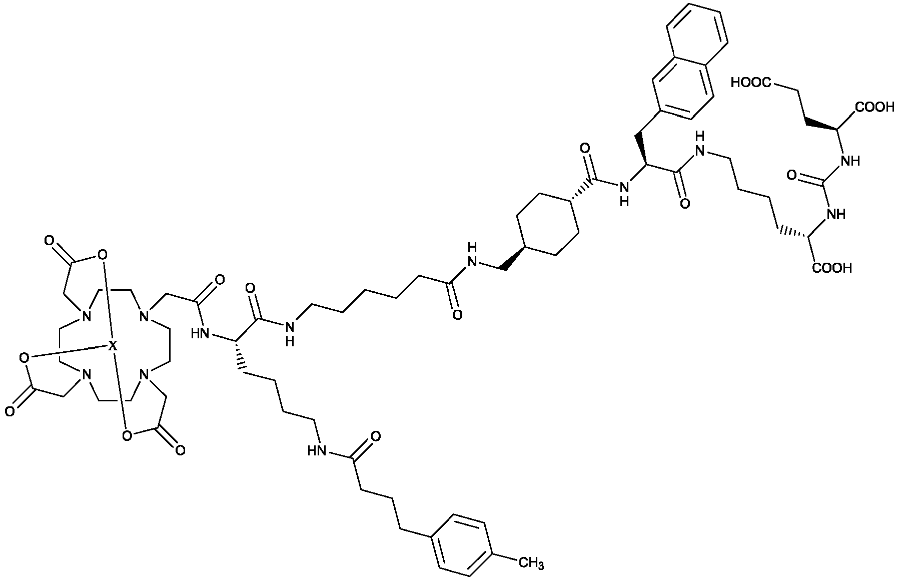

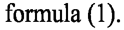

In an optional embodiment, the X may be a radionuclide and may be Lu-177, Ac-225, Cu-64 or Cu-67.

According to an embodiment of the present invention, the radionuclide complex of the present invention is labeled with Ac-225 and includes a structure of a compound of formula (3):

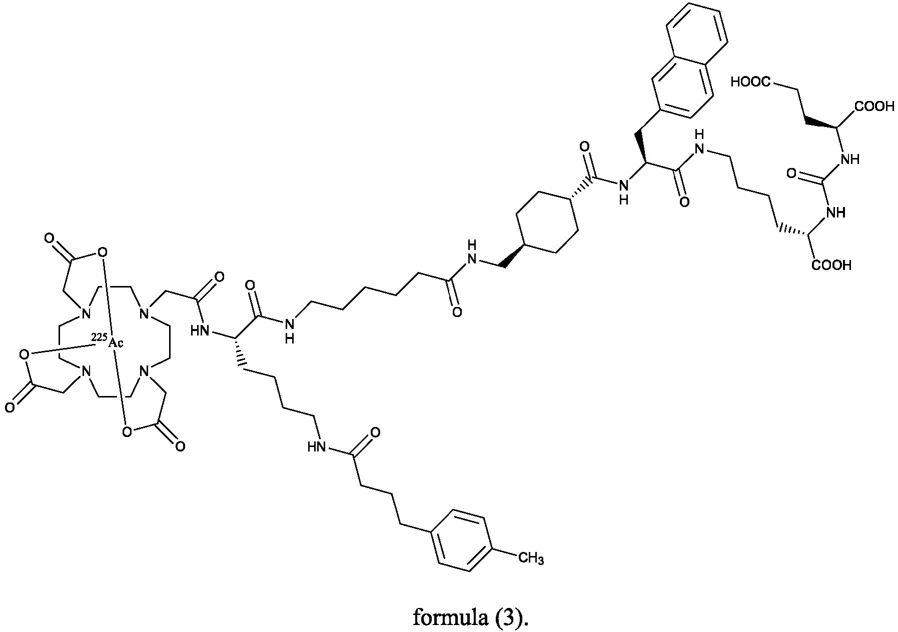

According to an embodiment of the present invention, the radionuclide complex of the present invention is labeled with Lu-177 and includes a structure of a compound of formula (4):

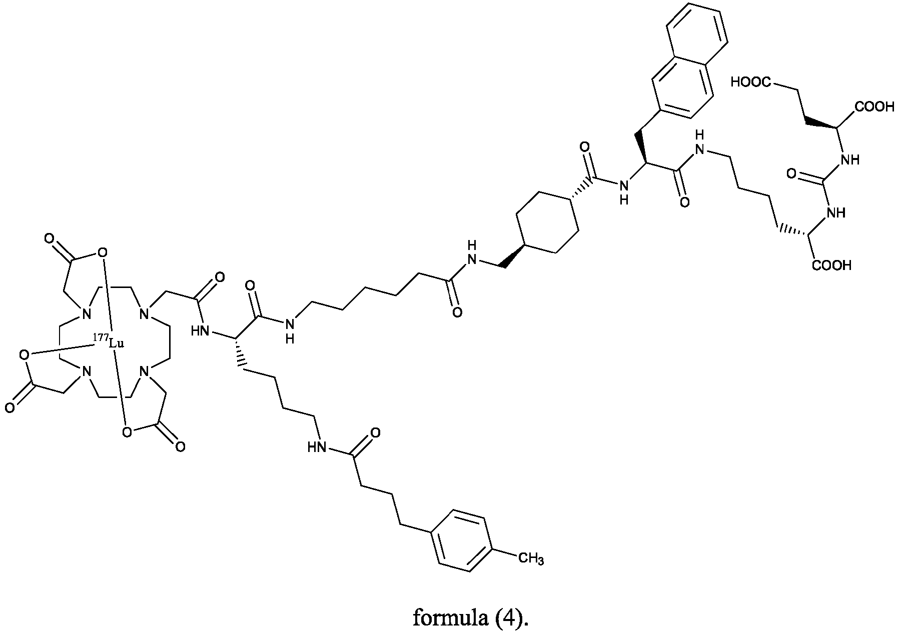

According to an embodiment of the present invention, the radionuclide complex of the present invention is labeled with Cu-64 and includes a structure of a compound of formula (5):

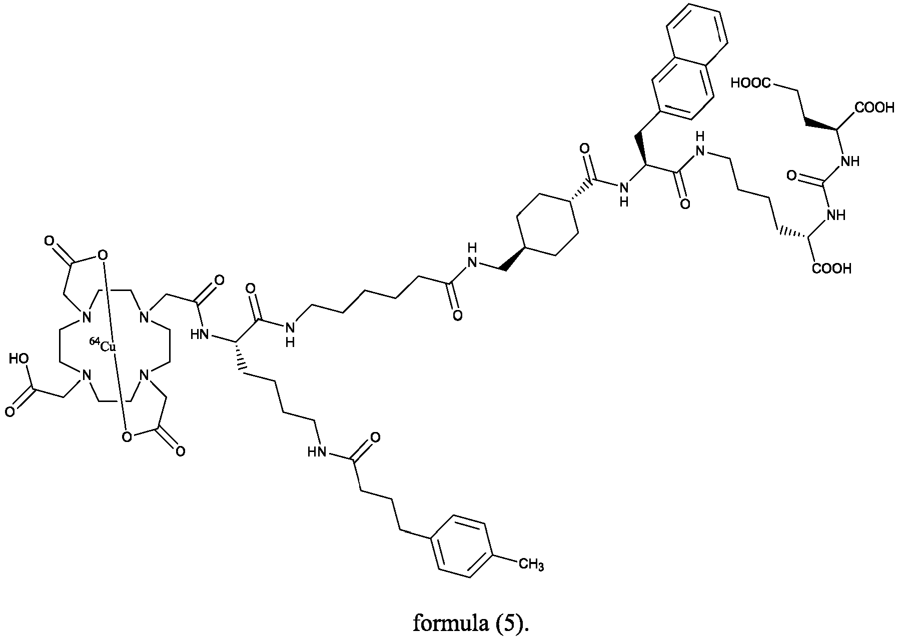

According to an embodiment of the present invention, the radionuclide complex of the present invention is labeled with Cu-67 and includes a structure of a compound of formula (6):

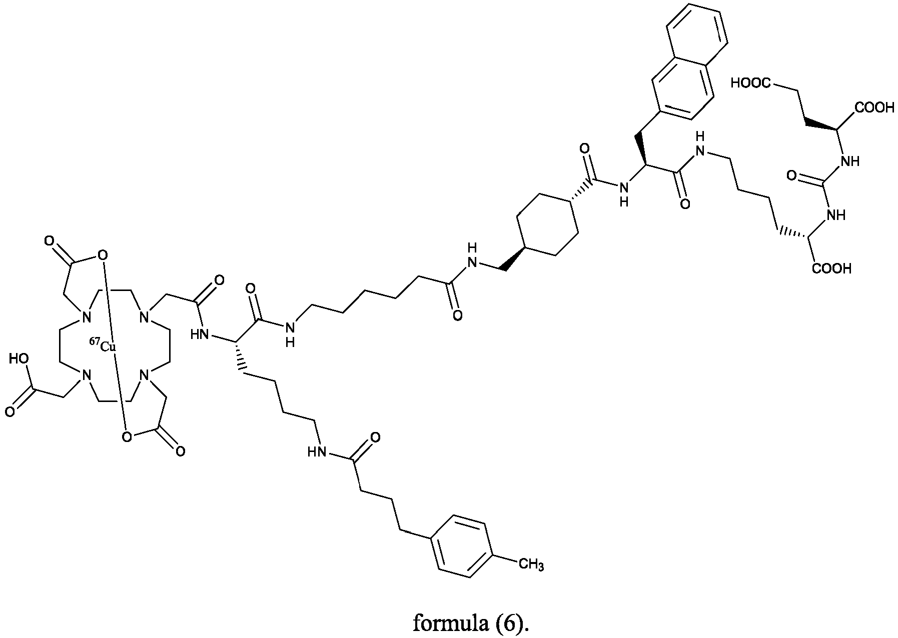

Those having ordinary skill in the art should understand that the activity of the radionuclide added to the mixed solution may be determined according to different types of radionuclides. For example, the radionuclide of the present invention may be Lu-177 (100-200 mCi); Ac-225 (0.1-0.4 mCi); Cu-64 (5-20 mCi); or Cu-67 (100-200 mCi). According to other embodiments of the present invention, the radionuclide is Lu-177 (130-150 mCi); Ac-225 (0.1-10.7 mCi); Cu-64 (5.0-20.0 mCi); or Cu-67 (60-75 mCi).

Those having ordinary skill in the art to which the present invention belongs should understand that appropriate radionuclides and activity may be selected according to different processes and actual usage requirements.

A plurality of examples are disclosed hereinafter to illustrate various implementation aspects of the present invention, so that those having ordinary skill in the art to which the present invention belongs can implement the technical content disclosed by the present invention according to the disclosure in this specification. Therefore, the examples disclosed below shall not be used to limit the scope of rights of the present invention. Furthermore, all the documents cited in this specification are regarded as becoming part of this specification by reference in their entirety.

### Example 1 Preparation of the radionuclide complex of the present invention using an auto-synthesizer

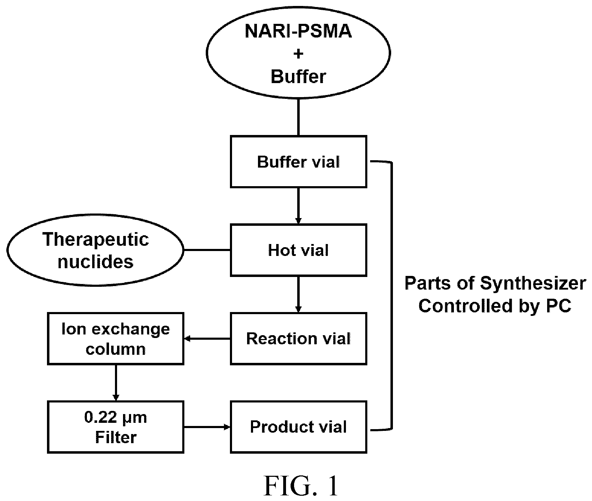

Please also refer to FIG. 1. This figure is a flowchart of preparing the radionuclide complex of the present invention using an automatic synthesis kit according to an embodiment of the present invention. Specifically, the components of the auto-synthesizer include: a hot vial, a buffer vial, a reaction vial, a product vial, a rinse vial, a computer-controlled connection device (including an air flow pushing device), a PC, an ion exchange column and a sterilization filter membrane (0.22 µm filter). The auto-synthesizer may be a commercially available Modular-Lab eazy synthesis cassette system.

First, an ascorbic acid buffer solution was prepared. 50 mg of ascorbic acid and 7.9 mg of sodium hydroxide were mixed and made up to 1 mL with water for injection (final concentration: ascorbic acid 0.284 M; sodium hydroxide 0.198 M). 1 mL of the ascorbic acid buffer solution was thoroughly mixed with 500 µg of NARI-PSMA (the compound of formula (2)) (20 mg/mL in DMSO or EtOH) (about 0.014 M of NARI-PSMA) and then added into the buffer vial of the auto-synthesizer. Next, the therapeutic radionuclide Lu-177 (200 mCi) or Ac-225 was added into the hot vial of the auto-synthesizer, and then automatic radiolabeling synthesis was started using the PC.

During the automatic radiolabeling synthesis, the therapeutic radionuclide was driven by gas and sent to the reaction vial. Then the solution in the buffer vial was first driven by gas into the hot vial and then into the reaction vial. Next, a reaction took place in the reaction vial heated at 95°C for 30 minutes. Subsequently, the solution in the rinse vial (using water for injection or normal saline) was brought by gas into the reaction vial where the reaction was completed. Then the solution in the reaction vial was pushed by gas through the ion exchange column and the sterilization filter membrane (0.22 µm filter) and then into the product vial. Finally, the radiochemical purity of the radionuclide complex of the present invention (Lu-177-NARI-PSMA or Ac-225-NARI-PSMA) was verified to be > 95% by isotope thin layer chromatography (iTLC) and radio-high pressure liquid chromatography (radio-HPLC).

### Example 2 Preparation of the radionuclide complex of the present invention in the form of a lyophilized formulation (freeze-dried formulation)

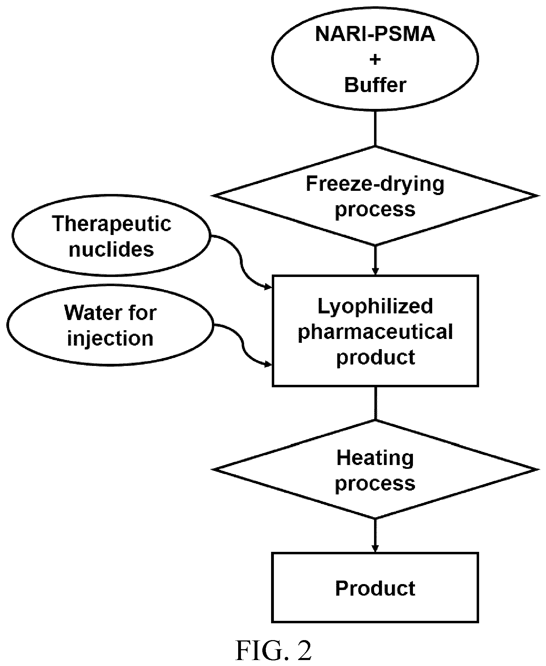

Please also refer to FIG. 2. This figure is a flowchart of preparing the radionuclide complex of formula (1) in the form of a lyophilized formulation according to an embodiment of the present invention. An ascorbic acid buffer solution (127 mg/mL) was prepared by using a sodium hydroxide solution (0.5 N) and ascorbic acid. Then the buffer solution was thoroughly mixed with NARI-PSMA (20 mg/mL EtOH) to form a bulk solution (NARI-PSMA: 0.014 M; ascorbic acid: 0.721 M). Next, the bulk solution was filtered through a sterilization filter membrane (0.22 µm filter), and the filtrate was dispensed into 20 mL colorless Type I glass bottles with 2 mL per bottle. Subsequently, the glass bottles containing 2 mL of the bulk solution underwent a freeze-drying step to obtain the lyophilized formulation of the radionuclide complex of formula (1).

Before use, the water for injection and the therapeutic radionuclide Lu-177 (130.0-150.0 mCi) or Ac-225 (0.1-0.4 mCi), Cu-64 (5.0-20.0 mCi) or Cu-67 (60.0-75.0 mCi) were added to the lyophilized formulation, and then thoroughly shaken and mixed to completely dissolve the lyophilized cake. Next, the mixture was placed in a heater (95°C, 30 minutes) or a sterilization autoclave (121°C, 20 minutes) for heating the reaction to obtain the radionuclide complex of the present invention (Lu-177-NARI-PSMA or Ac-225-NARI-PSMA).

### Example 3 Preparation of the radionuclide complex of the present invention using a sterilization autoclave (autoclave)

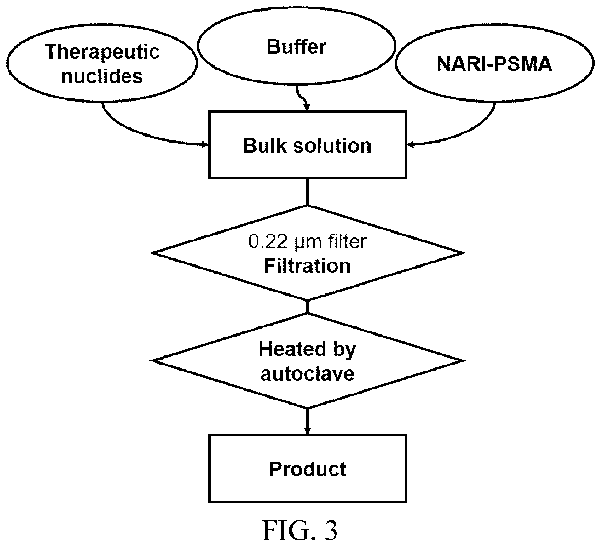

FIG. 3 is a flowchart of preparing the radionuclide complex of formula (1) using a sterilization autoclave according to an embodiment of the present invention. An ascorbic acid buffer solution, NARI-PSMA (20 mg/mL EtOH) and the therapeutic radionuclide Lu-177 (900 mCi) or Ac-225 were thoroughly mixed to form a bulk solution. Next, the bulk solution was filtered through a sterilization filter membrane (0.22 µm filter), and the filtrate was dispensed into 20 mL colorless Type I glass bottles with 2 mL per bottle. Subsequently, the glass bottles containing 2 mL of the filtrate (NARI-PSMA 107 µg; ascorbic acid 64.1 mg; Lu-177 with a radioactivity of 134-143 mCi or Ac-225 with a radioactivity of 1.07-10.7 mCi) were placed in a sterilization autoclave (121°C, 20 minutes) for heating the reaction to obtain the radionuclide complex of the present invention, i.e. Lu-177-NARI-PSMA or Ac-225-NARI-PSMA.

## Claims

1. A method for preparing a radionuclide complex, wherein the radionuclide complex has a structure as represented by formula (1): 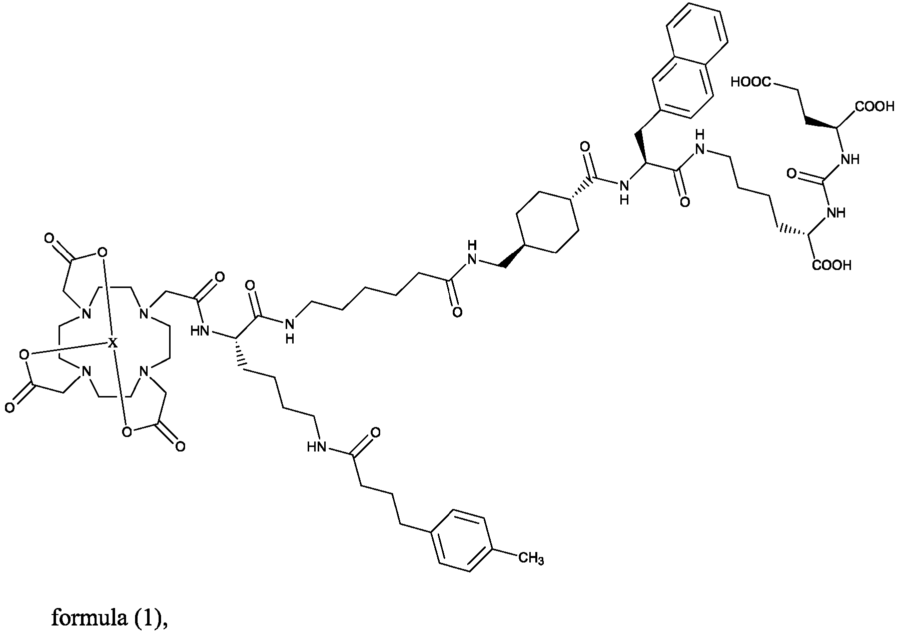 and the method comprises:
(a) mixing NARI-PSMA and an ascorbic acid buffer solution to form a mixed solution, wherein the NARI-PSMA is as represented by a compound of formula (2): 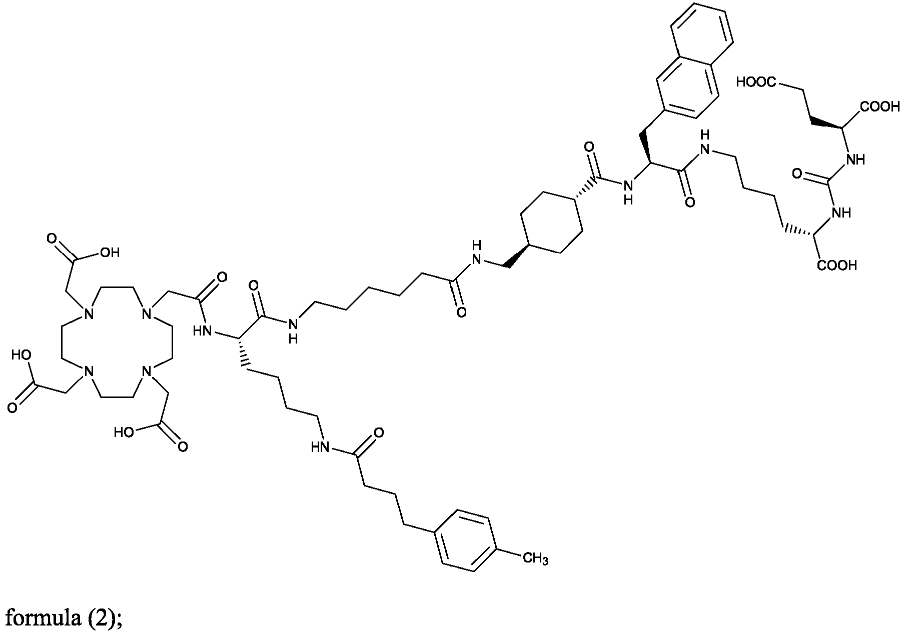
(b) adding a radionuclide to the mixed solution to obtain a mixed solution containing the radionuclide; and
(c) heating the mixed solution containing the radionuclide at 90°C-130°C for at least 10-30 minutes to obtain the radionuclide complex.

2. The method according to claim 1, wherein a concentration of the NARI-PSMA in the mixed solution is 0.0315-0.049 M.

3. The method according to claim 1, wherein X in the compound of formula (1) is the radionuclide, and the radionuclide is Lu-177, Ac-225, Cu-64 or Cu-67.

4. The method according to claim 1, wherein the step (c) further comprises:
filtering the mixed solution using a filter membrane.

5. The method according to claim 4, wherein the step (c) further comprises:
purifying the mixed solution using an ion exchange column.

6. The method according to claim 1, wherein the mixed solution in the step (a) is freeze-dried to obtain a lyophilized formulation, and in the step (b), water and the radionuclide are added to the mixed solution to obtain the mixed solution containing the radionuclide.

7. The method according to claim 1, wherein the ascorbic acid buffer solution has a pH value of 4.0-5.0.

8. The method according to claim 7, wherein a concentration of ascorbic acid in the ascorbic acid buffer solution is 0.02-0.72 M.

9. The method according to claim 1, wherein the method is capable of being performed using an auto-synthesizer.

10. The method according to claim 1, wherein in the step (c), the heating is performed by a sterilization autoclave.

11. The method according to claim 1, wherein in the step (c), the mixed solution containing the radionuclide is heated to 121°C for 30 minutes to obtain the radionuclide complex.
